# EUROPÄISCHE PATENTSCHRIFT

(11) **EP 1 177 305 B1**
(45) Veröffentlichungstag und Bekanntmachung des Hinweises auf die Patenterteilung: **26.12.2007**
(21) Anmeldenummer: 00931125.9
(22) Anmeldetag: 05.05.2000
(51) Int. Cl.: C12N 15/67, C07K 14/47, C12N 9/12, C12N 1/15, G01N 33/50

(54) **VERFAHREN ZUM HERSTELLEN EINES REKOMBINANTEN PROTEINS**
METHOD FOR PRODUCING A RECOMBINANT PROTEIN
PROCEDES DE PREPARATION D'UNE PROTEINE RECOMBINEE

(30) Priorität: 05.05.1999 DE 19920712
(43) Veröffentlichungstag der Anmeldung: 06.02.2002
(73) Patentinhaber: RHEIN BIOTECH GESELLSCHAFT FÜR NEUE BIOTECHNOLOGISCHE PROZESSE UND PRODUKTE MBH, 40595 Düsseldorf (DE); Gellissen, Gerd Prof. Dr., 42489 Wülfrath (DE)
(72) Erfinder: FARWICK, Mike, D-33615 Bielefeld (DE); LONDON, Markus, D-41541 Dormagen (DE); DOHMEN, Juergen, D-40670 Meerbusch (DE); DAHLEMS, Ulrike, D-47809 Krefeld (DE); GELLISSEN, Gerd, D-42489 Wuelfrath (DE); STRASSER, Alexander, W., D-40225 Duesseldorf (DE)
(74) Vertreter: Kahlhöfer, Hermann
(86) Internationale Anmeldenummer: PCT/EP2000/004062
(87) Internationale Veröffentlichungsnummer: WO 2000/068400

(56) Entgegenhaltungen:
- WO-A-98/31837
- US-A- 5 646 009
- US-A- 5 789 193
- PAUSCH M. H. ET AL.: "Multiple Ca2+/calmodulin-dependent protein kinase genes in a unicellular eukaryote." EMBO JOURNAL, Bd. 10, Nr. 6, 1991, Seiten 1511-1522, XP002146843 in der Anmeldung erwähnt
- MAYER A. F. ET AL.: "An expression system matures: A highly efficient and cost-effective process for phytase production by recombinant strains of Hansenula polymorpha." BIOTECHNOLOGY AND BIOENGINEERING, Bd. 63, Nr. 3, 5. Mai 1999 (1999-05-05), Seiten 373-381, XP002146844 in der Anmeldung erwähnt
- WEYDEMANN U. ET AL.: "HIGH-LEVEL SECRETION OF HIRUDIN BY HANSENULA POLYMORPHA-AUTHENTIC PROCESSING OF THREE DIFFERENT PREPROHIRUDINS" APPLIED MICROBIOLOGY AND BIOTECHNOLOGY, Bd. 44, Nr. 3-4, 1995, Seiten 377-385, XP000914759 ISSN: 0175-7598
- GELLISSEN G. ET AL.: "Heterologous gene expression in Hansenula polymorpha: efficient secretion of glucoamylase." BIOTECHNOLOGY, Bd. 9, März 1991 (1991-03), Seiten 291-295, XP002146845 in der Anmeldung erwähnt
- DOHMEN R. J. ET AL.: "Cloning of the Schwanniomyces occidentalis glucoamylase gene (GAM1) and its expression in Saccharomyces cervisiae." GENE, Bd. 95, 1990, Seiten 111-121, XP002146846 in der Anmeldung erwähnt
- WANG X. ET AL.: "The phosphorylation of eukaryotic initiation factor eIF4E in response to phorbol esters, cell stresses, and cytokines is mediated by distinct MAP kinase pathways." JOURNAL OF BIOLOGICAL CHEMISTRY, Bd. 273, Nr. 16, 17. April 1998 (1998-04-17), Seiten 9373-9377, XP002146847
- SODERLING T. R.: "The Ca(2+)-calmodulin-dependent protein kinase cascade." TIBS, Bd. 24, Juni 1999 (1999-06), Seiten 232-236, XP002146848
- CHOW TY ET AL.: "Screening and identification of a gene, PSE-1, that affects protein secretion in Saccharomyces cerevisiae" JOURNAL OF CELL SCIENCE, Bd. 101, Nr. 3, 1992, Seiten 709-719,
- OHYA ET AL.: "Two Yeast Genes Encoding Calmodulin-dependent Protein Kinases" THE JOUNAL OF BIOLOGICAL CHEMISTRY, Bd. 266, Nr. 19, 5. Juli 1991 (1991-07-05), Seiten 12784-12794,

## Beschreibung

Die vorliegende Erfindung bezieht sich auf Verfahren zum Herstellen von rekombinanten Proteinen, Insbesondere rekombinanten sekretorischen Proteinen.

Heutzutage wird eine Vielzahl von in Medikamenten enthaltenen aktiven Verbindungen nicht mehr durch chemische Synthese oder Gewinnung und Aufreinigung aus den natürlichen pflanzlichen oder tierischen Quellen hergestellt, sondern mit Hilfe rekombinanter DNA-Technologie erzeugt Diese Technologie bietet u.a. den Vorteil, daß die Proteine in gut charakterisierten Wirten in praktisch unbegrenzten Mengen erhalten werden können.

Für die Gewinnung von rekombinanten Proteinen ist es von Vorteil, wenn das betreffende Protein sezerniert wird, um anschließend aus dem Zellüberstand gewonnen werden zu können. Dies vereinfacht die Aufbereitung in hohem Maße, da das Protein bereits In relativ reiner Form vorliegt und die Anzahl aufwendiger Reinigungsschritte reduziert wer den kann

Häufig benutzte Organismen, die zur Gewinnung von sekretorischen Proteinen in großern Maßstab eingesetzt werden, sind Hefen und filamentöse Pilze. Sie bieten den Vorteil, daß sie relativ einfach in Zellkultur gehalten werden können und zu guten Ausbeuten an sezernierten Proteinen führen. Darüber hinaus sind sie in der Lage, die produzierten Proteine posttransiational zu modifizieren, z.B. zu glykosylieren.

Die Expression von rekombinanten DNA-Sequenzen wird im allgemeinen dadurch erreicht, daß Nukleinsäuresequenzen, die ein Protein von Interesse kodieren, in einen Vektor kloniert werden, der als funktionelle Elemente einen Promotor und Polyadenylierungssignale sowie Terminationselemente enthält. Dieser Vektor wird durch geeignete Verfahren, wie z.B. Elektroporation, Calcium- oder Liposomen-unterstützte Transfektion etc. in eine Wirtszelle inseriert Eine solche DNA-Sequenz kann weiterhin am 5'-Ende elne Sekretions-Leader-Sequenz enthalten, die veranlaßt, daß das rekombinante Translationsprodukt in den sekretorischen Apparat der Wirtszelle dirigiert wird.

Die Insertion von Fremd-DNA in ein solches Expressionssystem garantiert jedoch nicht eine wirksame Gen-Expression. Häufig auftretende Probleme sind eine ineffiziente Transkription und/oder Translation, rascher Abbau der Boten-RNA (mRNA) oder Instabilität des Protein-Produktes. Das rekombinante Protein kann außerdem inkorrekt und/oder ungenügend durch die Wirtszelle prozessiert und modifiziert werden. So werden z.B. in der Bäckerhefe Saccharomyces cerevisiae rekombinante Proteine oft sehr ineffizient sezerniert. Dieses Expressionssystem ist daher für viele Anwendungen unzulänglich. In solchen Fällen kann die Produktion jedoch unter Umständen durch die gleichzeitige Erzeugung weiterer Proteine, die eine effiziente Transkription und/oder Translation, Prozessierung, Stabilisierung, Modifikation und Verteilung in bestimmten Zellkompartimenten erlauben, verbessert werden. So wurden eine Reihe an S. cerevisiae-Mutanten hergestellt, die mögliche Engpässe in den von der Translation bis zur Sezernierung eines rekombinanten Proteins reichenden Prozessen ausräumen können. Derartige Mutationen sind von Smith et al. z.B. für die Gene ssc1 und ssc2 aus S. cerevisiae und für die Gene rgr1 und ose1 von Sakai et al. 1988, Hinnen et al., 1994 und von Gellissen und Hollenberg, 1997, beschrieben worden. Ein anderer Ansatz verwendet die Überexpression von heterologen Genen, die aus anderen Organismen eingeführt werden, wie z.B. die Überproduktion der Protein-Disulfid-Isomerase (PDI) oder des Chaperons BiP oder die Verwendung von Komponenten des Säugersignalpeptidasekomplexes in Bäckerhefe (Shusta et al. 1998; WO 98/13473).

Es ist bereits bekannt, daß methylotrophe Hefen, d.h. Hefen, die in der Lage sind, Methanol als einzige Energie- und Kohlenstoffquelle zu nutzen, rekombinante Proteine weniger stark hyperglykosylieren als S. cerevisiae. Des weiteren bieten methylotrophe Hefen den Vorteil, daß sie heterologe Proteine relativ effizient sezernieren. Das gleiche gilt auch z.B. für Kluyveromyces lactis, Aspergillus niger und Schizosaccharomyces pombe (Giga-Hama und Kumagai, 1997; Gellissen und Hollenberg, 1997; Hollenberg und Gellissen, 1997). Selbst die Verwendung von methylotrophen Hefen als Expressionswirten läßt jedoch die Produktion und Sezernierung nur einer begrenzten Anzahl von rekombinanten Proteinen zu. Es besteht daher ein großer Bedarf nach einem System, das die Möglichkeit bietet, jedes sekretorische Protein von Interesse effizient zu sezernieren. Darüber hinaus werden Verfahren benötigt, die es zulassen, rekombinante Proteine mit weitgehend korrekten sekundären Modifikationen, d.h. Modifikationen, wie sie auch nach Produktion des Proteins in seiner natürlichen Wirtszelle beobachtet werden, zu erzeugen.

Es ist daher Aufgabe der vorliegenden Erfindung, einen Weg für die effiziente Sezernierung von rekombinanten Proteinen und gegebenenfalls für die Produktion und/oder Sezernierung von rekombinanten Proteinen mit sekundären Modifikationen zur Verfügung zu stellen, welche den im natürlichen Wirt erzeugten sekundären Modifikationen weitgehend ähnlich sind.

Erfindungsgemäß wird diese Aufgabe durch ein Verfahren mit den Merkmalen des Anspruchs 1.

Der eukaryontische Initiationsfaktor 4E (eIF4E) spielt für die Initiation der Translation eine wichtige Rolle. Er ist Bestandteil des "Cap-Binding"-Komplexes (eIF4F) und ist für die Bindung dieses Komplexes und die 5-Cap-Struktur der mRNA (Sonenberg, 1996) verantwortlich. Eukaryontische Ca²⁺/Calmodulin-abhängige Proteinkinasen (CaM-Kinasen) vom Typ II phosphorylieren einer Reihe verschiedener Zelproteine und sind dadurch an der Regulation des Energiestoffwechsels, des Zellzyklus' und der Ionenpermeabilität etc. beteiligt (Schulman, 1993).

Der Begriff "biologische Aktivität" des eukaryontischen Translationsinitiationsfaktors 4E und der Ca²⁺/Calmodulin-abhängigen Proteinkinase bezieht sich dabei auf die In der Literatur beschriebenen Aktivitäten der genannten Enzyme. Die biologische Aktivität von elF4E wird dabei bestimmt, wie in Altmann & Trachsel (1989) beschrieben, die von CaM-Kinase, wie In Ohya et al. (1991) beschrieben.

Der Promotor P1 kann jeder beliebige Promotor sein, der in der gewählten Wirtszelle als Promotor funktioniert Beispiele solcher Promotoren finden sich im Stand der Technik reichlich (s. z. B. Sambrook et al., 1989).

Das Verfahren gestattet es, durch die Coexpression der Gene für die Ca²⁺/Calmodulin-abhängige Proteinkinase bzw. Derivaten davon zusammen mit der Expression eines Genes für ein Protein von Interesse eine effiziente Produktion und Sezernierung des betreffenden Proteins zu erzielen.

Es wurde beobachtet, daß durch die starke Überproduktion eines rekombinanten Proteins oft die Transkriptions/Translations- und Sezemierungsmaschinerie der Wirtszelle überlastet ist, so daß sie ihr Wachstum verlangsamt oder gar einstellt. Ein häufiger Engpaß besteht bei der Initiation der Translation, bei der, wie von den Erfindern festgestellt, der ATP-abhängige Initiationsfaktor 4E limitierend wirken kann. Durch Coexpresslon des Genes für diesen Translationsinitiationsfaktor mit einer rekombinanten DNA-Sequenz kann dieser Engpaß behoben werden. Überraschenderweise wirkt sich die Coproduktion des Translationsinitiationsfaktors 4E jedoch auch auf die Sekretionseffizienz aus: die Erfinder konnten eine stark erhöhte Sezernierung des erwünschten sekretorischen Proteins beobachten, sobald dieses mit 4E coproduziert wurde. In der vorliegenden Erfindung wird darüber hinaus zum ersten Mal gezeigt, daß auch die gemeinsame Produktion (Pausch et al., 1991; Ohya et al., 1999) von CaM-Kinase mit einem rekombinanten Protein zu einer effizienten Sezernierung des entsprechenden rekombinanten Proteins führt Der Wirkmechanismus, der zu einer gesteigerten Proteinsezemierung führt, ist jedoch in beiden Fällen noch ungeklärt.

Das erfindungsgemäße Verfahren erlaubt somit, auch im Fall stark überproduzierter Proteine diese wirksam so sezernieren, so daß auch für sekretorische Proteine extrem hohe Ausbeuten erzielt werden können. Die Produktion kann dabei durch einzelne oder eine Kombination mehrerer Maßnahmen gesteigert worden sein. So kann bereits durch die Wahl des Vektors die Kopienzahl pro Zelle beeinflußt werden. Vektoren auf 2 µ-Basis liegen mit 5 bis über 100 Kopien pro Zelle vor; meistens sind pro Hefezelle 20 bis 40 Kopien vorhanden. Integrative Vektoren können sogar in 80 bis 150 Kopien pro Zelle vorhanden sein.

Weiter kann durch die Wahl des Selektionssystems Einfluß auf die Kopienzahl des Vektors genommen werden. Die Verwendung Tryptophan-auxotropher Mutanten, deren Auxotrophie durch den Selektionsmarker *TRP1* auf dem Plasmid komplementiert wird, führt zur Amplifizierung des Plasmides in der auxotrophen Zelle. Eine Möglichkeit, die Kopienzahl darüber hinaus zu steigern, bietet die Verwendung von *LEU2*-auxotrophen Stämmen und deren Komplementierung mit dem *Leu2d*-Allel. Das *Leu2d*-Allel ist ein *LEU2*-Gen mit weitgehend deletiertem Promotor und dementsprechend schwacher Transkription. Der dringende Bedarf der Zelle an LEU2-Protein führt zu einer extremen Amplifizierung des Plasmides einschließlich eines evtl. darauf lokalisierten Strukturgenes für ein gewünschtes rekombinantes Protein.

Ein zusätzlicher Faktor, der die Effizienz des Produktionssystemes betimmt, ist die Stärke des jeweils dem Strukturgen für das gewünschte zu exprimierende Gen vorangestellten Promotors P2. Dem Fachmann sind unterschiedlich starke, induzierbare und nicht induzierbare Promotoren bekannt. Siehe dazu das schon erwähnte Handbuch von Sambrook et al., in dem eine Vielzahl von Promotoren beschrieben ist. Für die Produktion in Hefe ist es bevorzugt, den *GAL1*-Promotor oder den *PDC1*-Promotor zu verwenden.

Die so hergestellten Proteine können leicht aus dem Zellüberstand gewonnen werden, indem dieser durch Zentrifugation von Zellrückständen befreit wird und durch geeignete Reinigungsverfahren, wie z.B. durch lonenaustauschchromatographie, Affinitätschromatographie, Gel-Elektrophorese, etc. weiter aufbereitet wird.

Unter einem "Homolog" sind solche Proteine zu verstehen, die eine Homologie von mindestens 60% zu der Aminosäuresequenz des Translationsinitiationsfaktors 4E aus Saccharomyces cerevisiae (Brenner et al., 1988) besitzen. In bevorzugten Ausführungsformen beträgt die Homologie 70% oder 80%, besonders bevorzugt sind 90% oder 95%. Am stärksten bevorzugt ist eine Homologie von 98% oder 99%. Die Homologie kann dabei berechnet werden, wie in Pearson und Lipman, 1988, dargestellt.

Der dem Fachmann bekannte Ausdruck "Homologie" bezeichnet den Grad der Verwandtschaft zwischen zwei oder mehr Proteinen, der durch die Übereinstimmung zwischen den Aminosäuresequenzen mittels bekannter Verfahren, z. B. der computergestützten Sequenzvergleiche (Basic local alignment search tool, S.F. Altschul et al., J. Mol. Biol. 215 (1990), 403-410) bestimmt wird. Der Prozentsatz der "Homologie" ergibt sich aus dem Prozentsatz identischer Bereiche in zwei oder mehr Sequenzen unter Berücksichtigung von Lücken oder anderen Sequenzbesonderheiten. In der Regel werden spezielle Computerprogramme mit Algorithmen eingesetzt, die den besonderen Anforderungen Rechnung tragen.

Bevorzugte Verfahren zur Bestimmung der Homologie erzeugen zunächst die größte Übereinstimmung zwischen den untersuchten Sequenzen. Computerprogramme zur Bestimmung der Homologie zwischen zwei Sequenzen umfassen, sind jedoch nicht eingeschränkt auf das GCG Programmpaket, einschließlich GAP (Devereux, J., et al., Nucleic Acids Research 12 (12) : 387 (1984); Genetics Computer Group University of Wisconsin, Madison, (WI)); BLASTP, BLASTIN, und FASTA (Altschul, S. et al., J. Mol. Biol. 215:403-410) (1999)). Das BLASTX Programm kann vom National Centre for Biotechnology Information (NCBI) und aus weiteren Quellen bezogen werden (BLAST Handbuch, Altschul S., et al., NCB NLM NIH Bethesda MD 20894; Altschul, S., et al., Mol. Biol. 215:403-410 (1990)). Auch der bekannte Smith Waterman-Algorithmus kann zur Bestimmung von Homologien verwendet werden.

Bevorzugte Parameter für den Aminosäuresequenz-Vergleich umfassen die nachstehenden:

| | |
|---|---|
| Algorithmus: | Needleman und Wunsch, J. Mol. Biol 48:443-453 (1970) |
| Vergleichsmatrix: | BLOSUM 62 aus Henikoff und Henikoff, PNAS USA 89(1992), 10915-10919 |
| Lücken-Wert (Gap Penalty): | 12 |
| Lückenlängen-Wert: | |
| (Gap Length Penalty): | 4 |

Homologie-Schwellenwert (Threshold of Similarity): 0

Das GAP-Programm ist auch zur Verwendung mit den vorstehenden Parametern geeignet. Die vorstehenden Parameter sind die Fehler-Parameter (default parameters) für Aminosäuresequenz-Vergleiche, wobei Lücken an den Enden den Homologie-Wert nicht verringern. Bei sehr kurzen Sequenzen im Vergleich zur ReferenzSequenz kann es weiterhin notwendig sein, den Erwartungswert auf bis zu 100.000 (expectation value) zu erhöhen und gegebenenfalls die Wortlänge (word size) auf bis zu 2 zu verkleinern.

Weitere beispielhafte Algorithmen, Lücken-Öffnungs-Werte (gap opening penalties), Lückenausdehnungs-Werte (gap extension penalties), Vergleichsmatrizen einschließlich der im Programm-Handbuch, Wisconsin-Paket. Version 9, September 1997, genannten, können verwendet werden. Die Auswahl wird von dem durchzuführenden Vergleich abhängen und weiterhin davon, ob der Vergleich zwischen Sequenzpaaren, wobei GAP oder Best Fit bevor zugt sind, oder zwischen einer Sequenz und einer umfangreichen Sequenz-Datenbank wobei FASTA oder BLAST bevorzugt sind, durchgeführt wird.

Eine mit dem oben genannten Algorithmus ermittelte Übereinstimmung von 60 % wird im Rahmen dieser Anmeldung als 60 % Homologie bezeichnet. Entsprechendes gilt für höhere Homologiegrade.

Die Aufgabe der Erfindung wird durch ein Verfahren zum Herstellen eines rekombinanten Proteins gelöst, weiches die folgenden Schritte umfaßt
a) Exprimieren einer für das rekombinante Protein kodierenden Nukleinsäure in einer geeigneten Wirtszelle zusammen mit einer für ein Polypeptid mit der biologischen Aktivität einer Ca²+ /Calmodulin-abhängigen Proteinkinase (CaM-Kinase) kodierenden Nukleinsäure, wobei die für ein Polypeptid mit der biologischen Aktivität von CaM-Kinase kodierende Nukleinsäure unter der Kontrolle eines Promotors P1 steht;
b) Gewinnen des Proteins.
   Durch die Coexpression des Genes für ein rekombinantes Protein zusammen mit dem Gen für eine ca²⁺/Calmodulin-abhängige Proteinkinase wird das Protein von Interesse effizient produziert und kann entweder aus dem Zellüberstand (im Falle eines sezemierbaren Proteins) oder aus dem Zellumen (im Falle eines nicht sezernierbaren, intrazellulären Proteins) gewonnen werden. Neben der effizienten Produktion bietet die Coexpression des Genes für ein gewünschtes Protein mit dem Gen für CaM-Kinase den großen Vorteil, daß die aus vielen Wirtszellen bekannte Hyperglykosylierung eines rekombinanten Proteins reduziert wird, d.h. das Glykosylierungsmuster des rekombinanten Proteins von Interesse ist dem Glykosylierungsmuster in seinem natürlichen Wirt wesentlich ähnlicher als z.B. dem Glykosylierungsmuster nach Produktion in S. cerevisiae ohne die Anwesenheit der überproduzierten CaM-Kinase.

In einer bevorzugten Ausführungsform dieses Verfahrens ist die Nukleinsäure, die für ein Polypeptid mit der biologischen Aktivität einer CaM-Kinase kodiert, ausgewählt aus der folgenden Gruppe:
i) einer Nukleinsäure mit der CMK2- Gensequenz , die für CaM-Kinase aus Saccharomyces cerevisiae kodiert (*CMK2*)*;*
ii) einer durch Degeneration des genetischen Codes, durch Deletion, Insertion und/oder Addition von der in i) genannten Nukleinsäure abgeleiteten Nukleinsäure mit der biologischen Aktivität einer CaM-Kinase;
iii) einem Fragment einer der in i) bis ii) genannten Nukleinsäuren, das für ein Polypeptid mit der biologischen Aktivität einer CaM-Kinase kodiert;

Geeignete Wirtszellen, in denen die rekombinanten Proteine der oben beschriebenen Verfahren produziert werden können, sind Pflanzenzellen, Tierzellen, Hefezellen, Pilz zellen oder Schleimpilzzellen.

In einer bevorzugten Ausführungsform sind die Terzellen Säuger- oder Insektenzellen.

Als Hefezellen sind Zellen der Gattungen Saccharomyces, Schizosaccharomyces, Kluyveromyces, Hansenula, Pichia, Schwanniomyces, Candida und Yarrowia wie z.B. Pichia pastoris, Pichia pinus, Kluyveromyces lactis, Hansenula polymorpha und Candida boidinii bevorzugt Die erfindungsgemäß ebenfalls bevorzugten Pilzzellen gehören den Gattungen Aspergillus, z.B. Aspergillus niger, Neurospara, Rhizopus und Trichoderma an. Eine als Expressionswirt besonders bevorzugte Schleimpilzzelle ist eine Zelle der Gattung Dictyostelium.

Gemäß der vorliegenden Erfindung findet die Expression der Gene für das Polypeptid und das Protein mit der biologischen Aktivität von CaM-Kinase unter der Kontrolle eines Promotors P1, der ein starker Promotor ist, statt

Dabei ist besonders bevorzugt, daß der Promotor P1 induzierbar ist Ein Beispiel für einen solchen Promotor ist der GAL1-Promotor (Johnston & Davis, 1984), der durch die beiden Zucker Glukose und Galaktose reguliert werden kann. Wenn das Medium, in oder auf dem die Wirtszellen wachsen, Glukose enthält, wird die Promotor-Aktivität von Genen, die unter der Kontrolle des *GAL1*-Promotors stehen, induziert, so daß relativ viel mRNA als Transkriptionsprodukt entsteht. In der Anwesenheit von Galaktose im Medium hingegen ist die Promotor-Aktivität nur sehr gering, was bedeutet, daß nur relativ wenige mRNA-Moleküle hergestellt werden.

In einer Ausführungsform der vorliegenden Erfindung ist das sekretorische Protein das Enzym Phytase. Phytase ist ein pflanzliches Enzym, das Myo-Inoslt aus Phytinsäure abspaltet. Myo-Inosit wird aufgrund seiner lipotrophen Wirkung medizinisch in der Lebertherapie eingesetzt. Wenn Phytase dem Futter von monogastrischen Tieren (z.B. Geflügel und Schweinen) zugesetzt wird, ermöglicht es den Tieren, Phosphat effektiv aus der Nahrung aufzunehmen und reduziert gleichzeitig die Menge an ausgeschiedenem Phosphat Dies bedeutet, daß bei Phosphat-armen Diäten dem Futter weniger oder gar kein Phosphat künstlich zugesetzt werden muß und daß der anfallende Tiermist aufgrund des reduzierten Phosphatgehalts weniger umweltschädlich ist.

In weiteren Ausführungsformen werden amylolytische Enzyme, z. B. α-Amylase oder Glukoamylase, oder Hormone erzeugt. In bevorzugten Ausführungsformen wird das rekombinante Protein überproduziert. Wie bereits oben beschrieben, kann die Produktion durch mehrere Faktoren, wie verwendeter Vektor, Selektionssystem und Promotor, beeinflußt werden. Erfindungsgemäß ist bevorzugt, die Produktion unter die Kontrolle starker und/oder induzierbarer Promotoren zu stellen.

Ein weiterer Aspekt der vorliegenden Erfindung ist die Verwendung mindestens einer Nukleinsäure zum Steigern der Sekretion eines rekombinanten sekretorischen Proteins aus einer Wirtszelle, wobei diese Nukleinsäure ausgewählt ist aus der folgenden Gruppe:
i) einer Nukleinsäure mit der des CMK2- Gensequenz, die für CaM-Kinase aus Saccharomyces cerevisiae kodiert
ii) einer durch Degeneration des genetischen Codes, durch Deletion, Insertion und/oder Addition von der in i) genannten Nukleinsäure abgeleiteten Nukleinsäure, die für ein Polypeptid mit der biologischen Aktivität einer CaM-Kinase kodiert;
iii) einem Fragment einer der in i) bis ii) genannten Nukleinsäuren, das für ein Polypeptid mit der biologischen Aktivität einer CaM-Kinase kodiert.

Die Coexpression einer der oben aufgeführten Nukleinsäuren mit der für ein gewünschtes Protein kodierenden Nukleinsäure ermöglicht die effiziente Sezernierung des gewünschten Proteins.

Rekombinante Proteine, die aus Wirtsorganismen sezerniert werden, welche eine oder mehrere der oben genannten Nukleinsäuren beinhalten, bieten den Vorteil, daß das gewünschte Protein effizienter sezerniert wird und damit einfacher aufgereinigt werden kann. Dadurch sinken die Produktionskosten. So gewonnene Proteine können z.B. als Medikamente, Nahrungsmittelzusätze oder in der Kosmetikindustrie verwendet werden.

In einer bevorzugten Ausführungsform liegen die oben erwähnten Nukleinsäuren (i) bis iii) in einen Vektor integriert vor. Dabei sollte der Vektor neben einem Promotor P1, der funktionell mit der betreffenden Nukleinsäure verbunden ist, Polyadenylierungs- und Translationsterminationssignale enthalten.

Weiterhin besonders bevorzugt ist, daß das sekretorische Marker-Protein das Enzym Glukoamylase (EC 3.2.1.3; Glukoamylase mit Debranching-Aktivität) Ist (Dohmen, 1990). In einer besonders bevorzugten Ausführungsform ist die für Glukoamylase kodierende Nukleinsäuresequenz in dem obengenannten Verfahren die *GAM1*-Sequenz aus S. occidentalis.

Weiterhin ist eine Wirtszelle von der vorliegenden Erfindung umfaßt, die eine durch ein rekombinantes Verfahren in die Zelle eingeschleuste Nukleinsäure in ihrem Chromosom enthält, welche aus der folgenden Gruppe ausgewählt ist:
i) einer Nukleinsäure mit der CMK2- Gensequenz, die für CaM-Kinase aus Saccharomyces cerevisiae kodiert (*CMK*2);
ii) einer durch Degeneration des genetischen Codes, durch Deletion, Insertion und/oder tion von der in i) genannten Nukleinsäure abgeleiteten Nukleinsäure, die für ein Polypeptid mit der biologischen Aktivität einer CaM-Kinase kodiert:
iii) einem Fragment einer der in i) bis ii) genannten Nukleinsäuren, das für ein Polypeptid mit der biologischen Aktivität einer CaM-Kinase kodiert;

Des weiteren umfaßt die Erfindung einen Kit, wie in Anspruch 11 definiert der eine zur Sezernierung und/oder Glykosylierung von Proteinen geeignete Wirtszelle und einen Expressionsvektor enthält, wobei der Expressionsvektor in funktioneller Verbindung mit einem Promotor eine Nukleinsäure umfaßt, die für ein Polypeptid mit der biologischen Aktivität einer CaM-Kinase kodiert und aus der folgenden Gruppe ausgewählt ist:
i) einer Nukleinsäure mit der CMK2- Gensequenz, die für CaM-Kinase aus Saccharomyces cerevisiae kodiert (*CMK*2);
ii) einer durch Degeneration des genetischen Codes, durch Deletion, Insertion und/oder Addition von der in i) genannten Nukleinsäure abgeleiteten Nukleinsäure, die für ein Polypeptid mit der biologischen Aktivität einer CaM-Kinase kodiert;
iii) einem Fragment einer der in i) bis ii) genannten Nukleinsäuren, das für ein Fragment mit der biologischen Aktivität einer CaM-Kinase kodiert;

Dieser Kit bletet dem Anwender den Vorteil, daß er im Falle eines sezernierbaren Proteins neben einer effizienten Sezernierung dieses Proteins auch gleichzeitig eine Glykosylierung des rekombinanten Proteins erzielen kann, die dem Glykosylierungsmuster in der natürlichen eukaryontischen Wirtszelle des Proteins stark ähnelt, d.h. eine häufig auf tretende Hyperglykosylierung vermieden bzw. reduziert wird.

Die Erfindung enthält weiterhin einen Kit, wie in Anspruch 10 definiert, der eine zur Sezernierung und/oder Glykosylierung von Proteinen geeignete Wirtszelle sowie einen Expressionsvektor enthält der in funktioneller Verbindung mit dem entsprechenden Promotor Nukleinsäuren umfaßt, die für Polypeptide mit der biologischen Aktivität einer CaM-Kinase und eines eIF4E kodieren und aus der folgenden Gruppe ausgewählt sind:
i) einer Nukleinsäure mit der CDC33. Gensequenz, die für elF4E aus Saccharomyces cerevisiae kodiert (*CDC33*);
ii) einer Nukleinsäure, die für ein Homolog von elF4E mit einer Homologie von mindestens 90% und mit der biologischen Aktivität von elF4E kodiert;
iii) einer durch Degeneration des genetischen Codes, durch Deletion, Insertion und/oder Addition von der in i) oder ii) genannten Nukleinsäure abgeleiteten Nukleinsäure, die für ein Polypeptid mit der biologischen Aktivität von elF4E kodiert;
iv) einem Fragment einer der in i) bis iii) genannten Nukleinsäuren, das für ein Polypeptid mit der biologischen Aktivität eines elF4E kodiert;
v) einer Nukleinsäure mit der CMK2-Gensequenz, die für CaM-Kinase aus Saccharomyces cerevisiae kodiert (*CMK2*);

ii) einer durch Degeneration des genetischen Codes, durch Deletion, insertion und/oder Addition von der in i) genannten Nukleinsäure abgeleiteten Nukleinsäure, die für ein Polypeptid mit der biologischen Aktivität einer CaM-Kinase kodiert;
iii) einem Fragment einer der in i) bis ii) genannten Nukleinsäuren, das für ein Polypeptid mit der biologischen Aktivität einer CaM-Kinase kodiert;

In einer weiteren Ausführungsform der vorliegenden Erfindung enthält ein erfindungsgemäßer Kit weiterhin einen leeren Expressionsvektor, der zum Hineinklonieren einer Nukleinsäure geeignet ist, welche ein rekombinantes und/oder rekombinantes sezernier bares Protein kodiert. Diese Kit-Ausstattung soll die einfache Handhabung für einen Anwender ermöglichen, der eine bestimmte Nukleinsäure schnell und sicher in einem geeigneten Wirt exprimieren möchte, um eine effiziente Sezernierung und/oder reduzierte Hyperglykosylierung des entsprechenden rekombinanten Proteins zu erzielen.

Des weiteren wird von der vorliegenden Erfindung ebenfalls ein Kit zur Verfügung gestellt der eine wie oben erwähnte Wirtszelle enthält, welche die zur Steigerung der Sezernierung notwendigen Sequenzen nicht auf einem Vektor, sondern In ihrem Genom trägt, in Verbindung mit einem leeren Expressionsvektor, der zum Hineinklonieren einer Nukleinsäure geeignet ist, welche ein rekombinantes und/oder rekombinantes sezernierbares Protein kodiert Dieser Kt erlaubt es, durch Transformation der ebenfalls vom Kit zur Verfügung gestellten Wirtszelle mit dem Expressionsvektor die effektive Produktion und gegebenenfalls Sezernierung eines Proteins von Interesse zu erzielen.

Die folgenden Abbildungen und Beispiele sollen dazu dienen, die verschiedenen Aspekte der vorliegenden Erfindung zu beschreiben und zu erläutern. Die Auswahl der genutzten genetischen Komponenten und DNA-Sequenzen soll das Grundprinzip der Erfindung veranschaulichen. In diesem Sinne werden die aufgeführten Beispiele nicht als beschränkend aufgefaßt.

### Abbildungen:

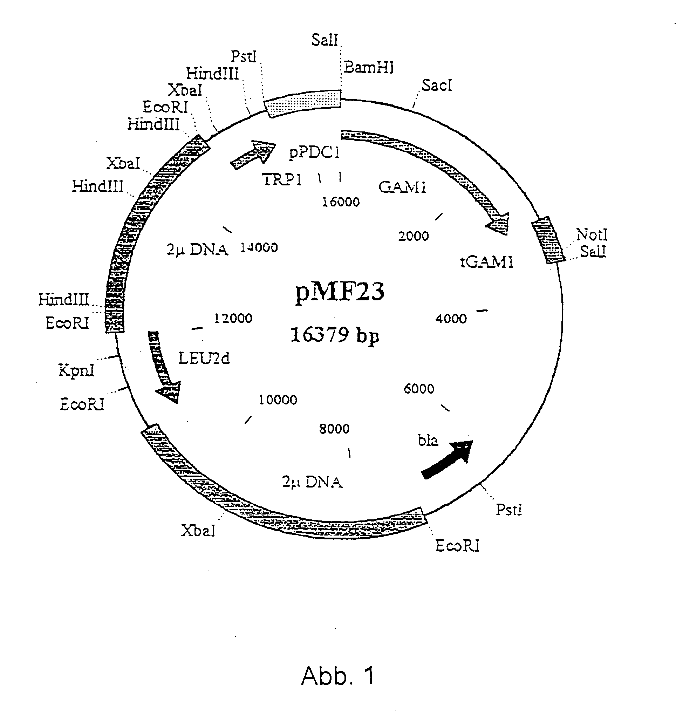

Abbildung 1 zeigt eine Plasmidkarte des Plasmids pMF23. Das Plasmid ist ein E.coli/S. cerevisiae Shuttle-Vektor. Es trägt die folgenden funktionellen Einheiten: einen Replikationsursprung (oriR) und das β-Lactamase-Gen (bla) zur Propagierung und Selektion in E.coli und die gesamte 2 µ-DNA für die Propagierung in S. cerevisiae. Markersequenzen für die Selektion in Hefe sind das *TRP1-*Gen und das *LEU2d*-Allel. Das Plasmid pMF23 enthält das von S. occidentalis stammende GAM1-Gen einschließlich des Terminators unter der Kontrolle des aus S. cerevisiae stammenden *PDC1*-Promotors.

pMF25 ist wie pMF23 aufgebaut, enthält jedoch bei im wesentlichen gleicher kodierender Sequenz ein C-terminales Hämagglutinin-Epitop aus einem Influenzavirus.

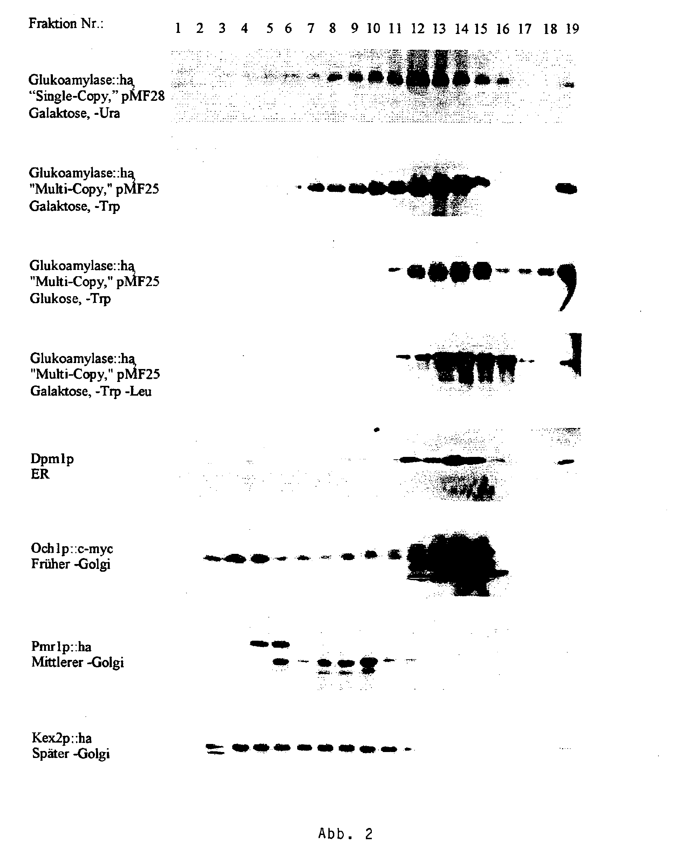

Abbildung 2 zeigt die subzelluläre Lokalisation von Gam1p-Protein in verschiedenen S. cerevisiae-Stämmen in Abhängigkeit von der Expressionsstärke. Das GAM1-Gen wurde entweder von dem "single copy plasmid" pMF28 oder dem "multi copy plasmid" pMF25 exprimiert. Das Plasmid pMF28 ist ein CEN-Vektor, der das *URA3*⁺-Gen als Selektionsmarker und die mit einer für das Hämagglutinin kodierenden Sequenz fusionierte GAM1-Sequenz enthält. Die Zellen wurden in 100 ml Minimalmedium, ohne Uracil (pMF28) oder ohne Tryptophan (pMF25), mit Galaktose oder Glukose (wie angegeben) als einziger Kohlenstoffquelle herangezogen oder sie wurden für 30 Stunden in einem Leucin-freien Minimalmedium in ihrem Wachstum arretiert. Zellfraktionen wurden durch Zentrifugation mit einem linearen Saccharosegradienten (7-47%) aufgetrennt und auf ihrem Gehalt an Gam1p-Protein durch SDS-PAGE und Western Blot unter Verwendung von spezifischen Anti-Hämagglutinin-Antikörpern untersucht. Die subzellulären Kompartimente wurden durch die Anwesenheit von spezifischen Marker-Proteinen identifiziert.

Als Marker-Proteine wurden Dolichol-Phosphat-Mannose-Syntase (Dpm1p - Endoplasmatisches Retikulum), Oligosaccharyltransferase (Och1p - früher Golgi), ATPase (Pmr1 p - medialer Golgi), Endoprotease (Kex2p - später Golgi) verwendet.

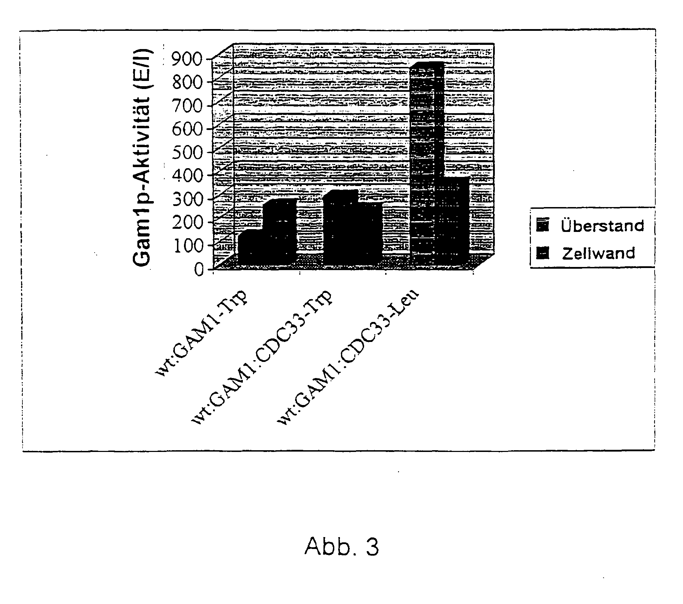

Abbildung 3 zeigt die im Kulturüberstand und der Zellwandfraktion von S. cerevisiae-Stämmen bestimmte Gam1 p-Aktivität. Die Zellen wurden in 20 ml SG-Medium ohne Tryptophan oder ohneTryptophan und ohne Leucin, inokuliert. Supertransformanden, die zusätzlich Plasmid mit der für *CDC33* kodierenden DNA enthielten, wurden ohne Uracil kultiviert. Die Zellen wurden bis zur stationären Phase wachsen gelassen, und dann die Glukoamylase-Aktivität, wie in Beispiel 3 beschrieben, bestimmt. Die gemessenen Aktivitäten sind Durchschnittswerte von mindestens drei unabhängigen Bestimmungen.

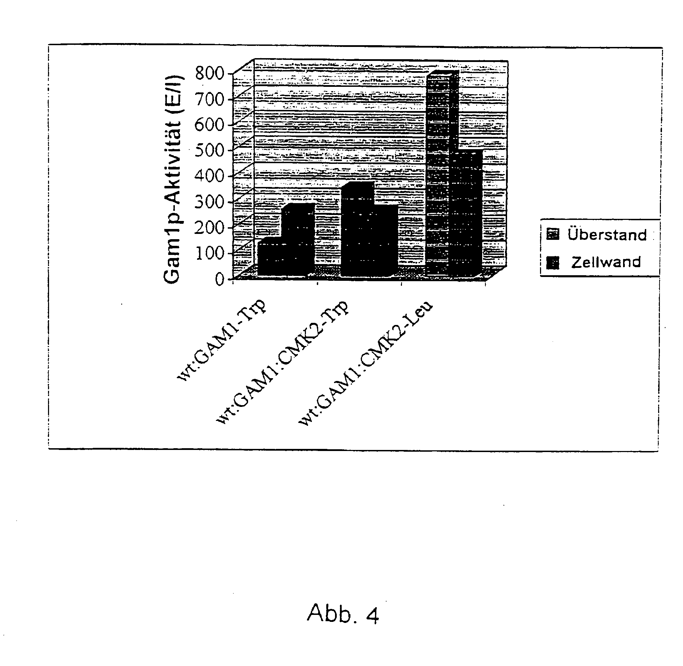

Abbildung 4 zeigt die Bestimmung der Gam1 p-Aktivität im Kulturüberstand und der Zellwandfraktion von S. cerevisiae-Stämmen. Alle Bedingungen waren mit den in Abbildung 3 beschrieben Bedingungen identisch. Als Vergleich wurde ein Stamm benutzt, der mit dem für *CMK*2 kodierenden Plasmid supertransformiert war.

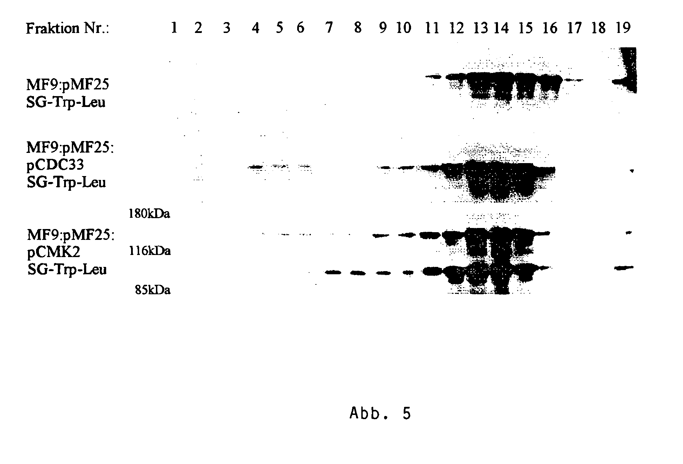

Abbildung 5 zeigt das Ausmaß der Glykosylierung von Gam1p in *CMK2-* und *CDC33-*überexprimierenden S. cerevisiae-Stämmen. Die Stämme wurden wie in Abb. 2 beschrieben kultiviert und subzelluläre Fraktionen auf ihren Gehalt an Gam1p überprüft. In CMK2-überexprimierenden Stämmen ist ein großer Anteil des Gam1 p-Protein nicht hyperglykosyliert.

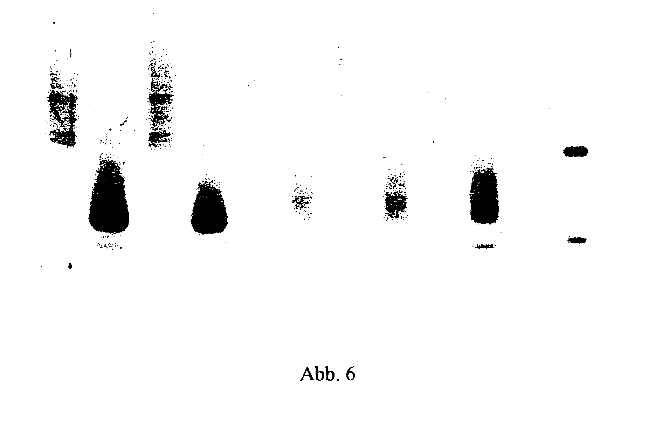

Abbildung 6 zeigt das Ausmaß der Glykosylierung von Phytase in *CMK2*-überexprimierenden Hansenula polymorpha-Stämmen. H. polymorpha Zellen, die Phytase herstellten, wurden mit einem Plasmid supertransformiert, welche das von S. cerevisiae stammende CMK2-Gen unter der Kontrolle des *GAL1*-Promotors aus S. cerevisiae enthielten. Der Phytase herstellende Stamm und repräsentative Beispiele der Supertransformanden wurden in Glycerin-Medium (5 % Glycerin, 0,1 M P0₄, pH 5,0) kultiviert. Das sezernierte Phytase-Protein wurde mittels SDS-PAGE analysiert und mit dem sezernierten und durch Behandlung mit Endo H deglykosylierten Produkt verglichen. Die Abbildung zeigt Phytase-Protein, welches von dem ursprünglichen Phytase-Produktionsstamm sezerniert wird, im Vergleich zu Phytase, die von einem supertransformierten Stamm hergestellt wurde, welcher zusätzlich ein *GAL1*-Promotor-*CMK*2-Konstrukt trägt. In den Supertransformanden ist das Ausmaß an Hyperglykosylierung reduziert, so daß distinkte Polypeptidbanden beobachtet werden können.

Die Spuren 2, 4, 6, 8, 10 entsprechen Phytaseproben, die mit Endo H behandelt wurden; die Proben der Spuren 1, 3, 5, 7 und 9 wurden nicht vorbehandelt. Aufgetragen wurden in den Spuren
- 1 bis 4:: von supertransformierten Zellen sezernierte Phytase (zwei Kulturüberstände); in den Spuren
- 5 bis 10:: vom ursprünglichen Produktionsstamm sezernierte Phytase (drei Kulturüberstände), und in der Spur
- 11:: Molekulargewichtsstandard: die obere Bande entspricht 66,3 kDa, die untere Bande entspricht 55,4 kDa.

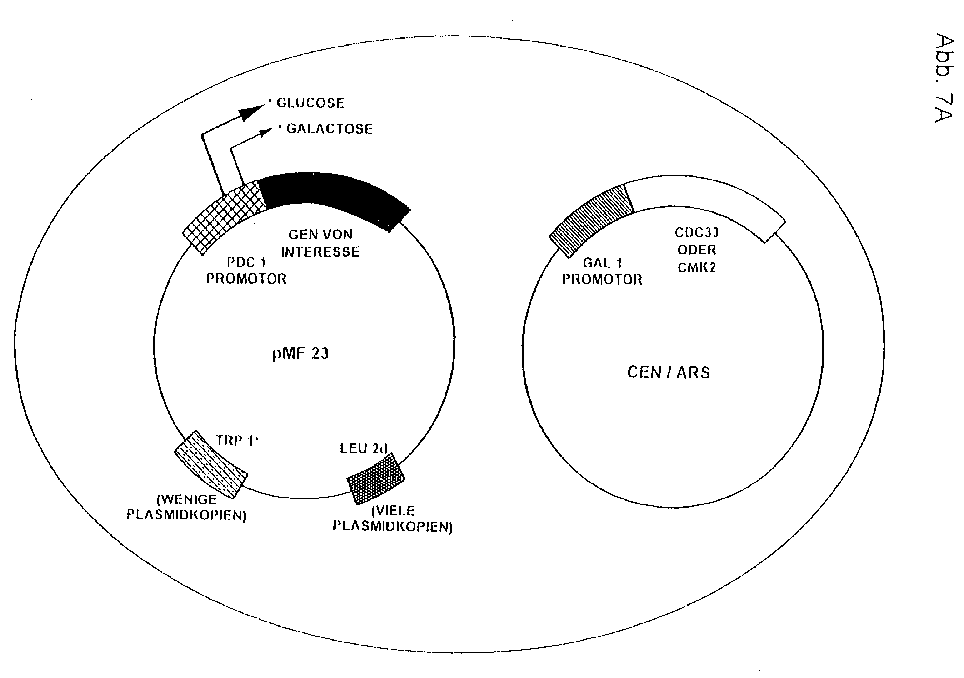

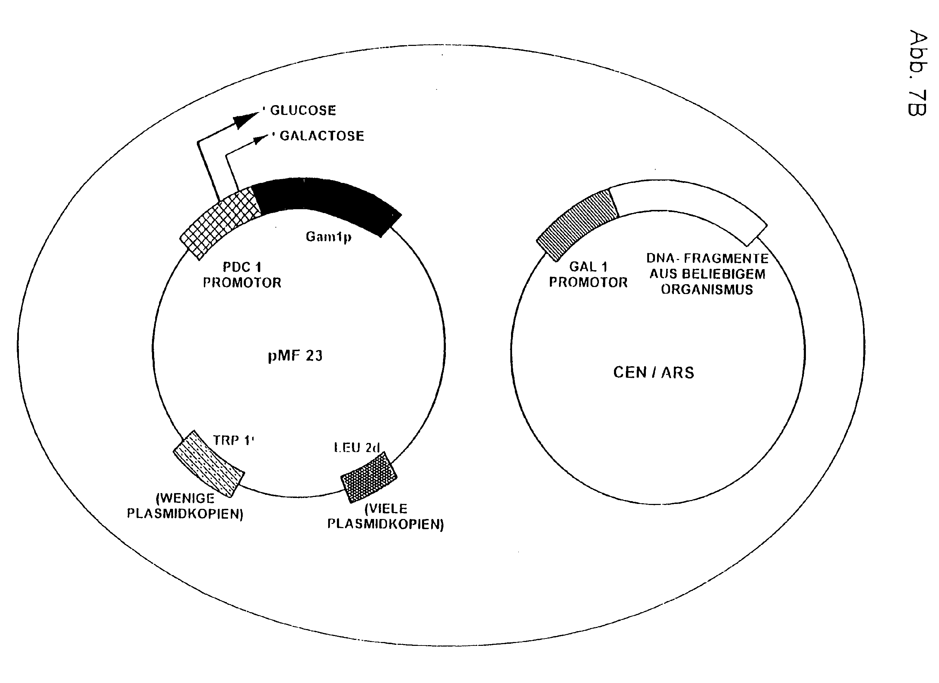

Abbildung 7 zeigt die Plasmid-Ausstattung einer Wirtszelle, die zur effizienten Sezernierung bzw. Reduktion der Hyperglykosylierung eines rekombinanten Proteins von Interesse geeignet ist (A) und einer Wirtszelle, die zum Identifizieren von Nukleinsäuremolekülen geeignet ist, welche eine Wachstumsinhibition des Wirtes dereprimieren können (B).

### Material und Methoden

| **Saccharomyces cerevisiaeStämme** | | |
|---|---|---|
| | | |
| **Stamm** | **Genotyp** | **Referenz** |
| JD52 | *MATα, leu2-3,112, trp1-Δ63, ura3-52, his3- Δ200, lys2-801, cir*⁺ | Dohmen *et al.,* 1995 |
| MF9 | *MATα, leu2-Δ1100, trp1-Δ63, ura3-52, his3-Δ200, lys2-801, cir°* | s. unten |

Im S. cerevisiae Stamm JD52 wurde durch homologe Rekombination mit einem funktionellen LEU2-Fragment der *LEU2*-ORF wieder hergestellt. Das funktionelle LEU2-Fragment wurde als *Sa*/l*-Xho*l Fragment aus pUC/LEU#5 erhalten. Das wiederhergestellte *LEU2*-Gen wurde anschließend im Bereich -407 bis +748 deletiert. Der resultierende Stamm wurde anschließend nach der Methode von Erhart und Hollenberg (1983) von der endogenen 2µ-DNA bereinigt, wodurch der Stamm MF9 entstand.

| **Plasmide** | | |
|---|---|---|
| **Plasmid** | **Konstruktion, enthaltene DNA-Sequenzen** | **Referenz** |
| pJDcPG-15 | CEN/ARS-Plasmid, enthält die *GAM1* Expressionskasette, *URA3* | Dohmen, 1989 |
| pJDaG-15 | *TRP1*/*ARS*-Plasmid, enthält die *GAM1* Expressionskasette | Dohmen, 1989 |
| pJDB219 | Gesamt 2µ-*LEU2d*-E.coli Shuttle-Vektor | Beggs, 1981 |
| pRS316 | CEN/ARS, *URA3* | Sikorski und Hieter, 1989 |
| pUC19 | E.coli-Klonierungsvektor | Sambrook *et al.,* 1989 |
| pUC/LEU#5 | pUC19 mit einem genomischen Sa*l*l-*Xho*l Fragment, welches das *LEU2*-Gen enthält | J. Dohmen, nicht publiziert |
| pMF23 | Aus pJDaG-15 wurde das ARS Element durch Bg*l*ll-Ps*t*l-Spaltung entfernt. Die kohäsiven Enden wurden durch Behandlung mit T4-Polymerase aufgefüllt und das Plasmid wurde religiert. In die *Eco*RI-Schnittstelle wurden die 2µ-*LEU*2*d*-Anteile aus pJDB219 ligiert, die durch partielle Spaltung dieses Plasmides mit *Eco*RI erhalten wurden. | diese Anmeldung, 2µ-*TRP1*/*LEU2d* Expressionsplasmid für *GAM1* unter der Kontrolle des *P_{PDC1}*-Promotors |
| pMF25 | Die PCR-Produkte MF238/246 (*GAM1*) und MF247/248 (*GAM1*-Terminator) wurden Sacl-*Kpn*I bzw. *Xba*I-*Not*I gespalten. Aus pJD307 wurde das HA₂-Epitop als *Kpn*I*-Xba*I Fragment (Dohmen *et al.,* 1995) erhalten und mit einem GAM1-*Sac*I-*Kpn*I-Fragment sowie einem *GAM1*-Terminator *Xba*I-*Not*I-Fragment in das mit *Sac*I/*Not*I geöffnete Plasmid pMF23 ligiert. | diese Anmeldung, wie pMF23; kodiert für eine C-terminal mit einem doppelten HA-Epitop versehene Glukoamylase |
| pMF28 | Umklonierung des *Sac*I/*Not*I Fragmentes aus pMF25 in pJDcPG-15 | diese Anmeldung, "*Single-Copy"-*Expressionsplasmid für *GAM1* |

### Methoden

### Medien

| Saccharomyces cerevisiae-Medien: | |
|---|---|
| Vollmedium YPD | 1 % Hefeextrakt; 2% Pepton; 2% Glukose |
| Vollmedium YPGal | 1 % Hefeextrakt; 2% Pepton; 2% Galaktose |
| Minimalmedium SD | 6,7 g/l Yeast Nitrogen Base w/o Aminosäuren; 2% Glukose; 50 mM Citrat pH 4,5 |
| Je nach Selektion wurden zusätzlich zugegeben | Histidin und Methionin je 0,01 g/l; Arginin 0,02 g/l; Lysin und Tryptophan 0,04 g/l; Threonin; 0,05 g/l; Leucin, Isoleucin und Phenylalanin 0,06 g/l; Uracil 40 mg/l, Adenin 20 mg/l |
| Minimalmedium SG | wie SD, jedoch 2% Galaktose statt 2% Glukose |

Feste Nährböden enthielten zusätzlich 1,8% Agar, gegebenenfalls wurde 0,5% lösliche Stärke nach Zulkowsky (Merck) zugegeben. Die Anzucht der Hefen erfolgte bei 30°C. wenn nicht anders vermerkt.

| Escherichia coli-Medien: | |
|---|---|
| LB-Medium | 1 % Trypton; 0,5% Hefeextrakt; 1 % NaCl |
| SOC-Medium | 2% Trypton, 0,5% Hefeextrakt; 20 mM Glukose,10 mM NaCl; 2,5 mM KCI; 10 mM MgCl₂; 10 mM MgSO₄; pH 7,5 |
| M9 Minimalmedium | Na₂HPO₄ x 7 H₂O 12,8 g/l, KH₂PO₄ 3 g/l, NaCl 0,5 g/l, NH₄Cl 1 g/l |

Feste Nährböden enthielten zusätzlich 1,8% Agar. Zur Selektion plasmidhaltiger Zellen wurde dem Medium 120 mg/l Ampicillin zugesetzt. Die Bakterien wurden bei einer Temperatur von 37°C kultiviert.

### Beispiel 1

### Herstellung eines Systems zum Identifizieren von Genen, die die Sekretion von heterologen Proteinen in einer S. cerevisiae Wirtszelle beeinflussen

Aus den Plasmiden pJDaG-15 und pJDB219 wurde ein 2µ Plasmidderivat, welches mit pMF23 bezeichnet wurde, hergestellt (Dohmen, 1989; Beggs, 1981). Das resultierende Plasmid ist in Abb. 1 dargestellt und enthält neben der gesamten 2 µ-DNA eine aus S. occidentalis abstammende *GAM1*-Sequenz, welche an ein *PDC1-*Promotorelement fusioniert ist. Ferner enthält das Plasmid die Selektionsmarker *TRP1* und das *LEU2d.* Bei *LEU2*d handelt es sich um ein Allel des *LEU2*-Gens, bei dem der Großteil des Promotors deletiert ist und das daher schwach exprimiert wird. Die Verwendung dieser beiden Selektionsmarker gestattet es, den Selektionsdruck zu variieren und die Kopienzahl der in den S. cerevisiae-Stamm MF9 (trp1, leu2, cir°) eingeführten Plasmide zu beeinflussen. Unter *LEU2d*-Selektionsbedingungen wird eine extrem hohe Kopienzahl des eingeführten Plasmids erhalten, während unter Selektionsbedingungen für *TRP1* eine zwar hohe, aber im Vergleich der *LEU2d*-Selektion geringere Kopienzahl erzielt wird. So enthalten rekombinante Stämme, die nach Transformation mit dem oben beschriebenen Plasmid erhalten wurden, unter *LEU2d*-Selektionsbedingungen relativ viele Kopien des *GAM1-*Gens, dessen Transkription durch den *PDC1*-Promotor reguliert wird. Wachstum auf Glukose-haltigem Medium erzielt eine hohe *PDC1*-Promotor-Aktivität, während Wachstum auf Galaktose zu einer geringeren Aktivität führt. Transformanden, die unter *LEU2d-*Selektionsbedingungen herangezogen wurden, sind weder in der Lage, auf Glukosenoch auf Galaktose-haltigem Medium zu wachsen. Ein Vergleich dieser Zellen mit Transformanden aus Kontrolltransformationen, bei denen Plasmide verwendet werden, die für enzymatisch inaktive Glukoamylase kodieren, zeigte, daß die unter *LEU2d-*Selektionsbedingungen beobachtete Wachstumsinhibition dadurch verursacht wurde, daß für das rekombinante Enzym ein zu hoher Sekretionsdruck bestand. Die Wachstumsinhibition wurde nicht durch die enzymatische Aktivität der Glukoamylase verursacht. Dies wurde durch die Beobachtung bestätigt, daß Gam1p-Produktion unter weniger stringenten Bedingungen zu einer Akkumulierung des Enzyms in dem Endoplasmatischen Retikulum und dem cis-Golgi führte (Abb. 2).

Derartige Transformanden wurden benutzt, um sie mit den klonierten DNA-Sequenzen der S. cerevisiae-cDNA Genbank zu retransformieren. Dazu wurde eine cDNA-Genbank aus S. cerevisiae verwendet, die unter der Kontrolle des *GAL1*-Promotors in einen CEN/ARS-Vektor kloniert wurde (Liu et al. 1992; Ausubel et al., 1987). Die S. cerevisiae-Stämme wurden entweder auf nicht selektivem Medium (YPD) oder auf einem Minimalmedium wachsen gelassen. Stämme, die die Fähigkeit, die Glukoamylase unter den oben beschriebenen stringenten Bedingungen zu sezemieren und dann zu wachsen, wiedererlangt hatten, hatten DNA-Fragmente aufgenommen, welche dazu führten, daß die beobachtete Wachstumsinhibition und damit die Limitierung der Sekretion überwunden werden konnten. Plasmide aus solchen Transformanden, die eine Suppression der Wachstumsinhibition des Gam1p-überproduzierenden Wirtsstammes zeigten, wurden durch DNA-Sequenzierung analysiert.

Eine Analyse des unter erfindungsgemäßen Bedingungen erhaltenen Glukoamylase-Proteins und sein Vergleich mit Standard-Isolaten , d.h. Isolaten, die unter Verwendung gleicher Randbedingungen mit der Ausnahme erhalten wurden, daß statt der *CMK2* und/oder CDC33 enthaltenden Vektoren entsprechende Leervektoren verwendet wurden, ermöglichten es weiterhin, sekundäre Modifikationen, wie z.B. das Glykosylierungsmuster des sezernierten Produktes, zu beurteilen.

### Beispiel 2

### Identifizierung von S. cerevisiae-Genen, die die Wachstumsinhibition in Gam1p überproduzierenden S. cerevisiae-Stämmen supprimieren

Der Stamm MF9: pMF23 wurde mit der oben beschriebenen cDNA-Genbank transformiert. 420.000 Transformanden wurden für zwei Tage auf SG-Platten ohne Tryptophan und Uracil wachsen gelassen. Die so erhaltenen Kolonien wurden auf SG-Platten ohne Leucin, Tryptophan und Uracil durch Replika-Plattierung repliziert. 320 Leucin-prototrophe Klone wurden erhalten, von denen 66 ein Wachstum in Abhängigkeit von der Kohlenstoffquelle (Wachstum auf Galaktose, kein Wachstum auf Glukose) zeigten. Alle erhaltenen Klone sezernierten Glukoamylase. Die cDNA-Inserts der 55 am besten wachsenden Klone wurden durch PCR-Ampiifikation analysiert. Acht verschiedene cDNA-Größen konnten unterschieden werden, wobei 3 Größen mehrmals vertreten waren. Insgesamt 13 Plasmide wurden isoliert und durch DNA-Sequenzierung analysiert. Die Fähigkeit der isolierten DNA-Sequenzen, eine Wachstumsinhibition zu dereprimieren, wurde durch Retransformation der einzelnen Sequenzen In den Stamm MF9:pMF23 bestätigt Die Identität der erhaltenen Sequenzen wurde durch Vergleich mit Daten aus Sequenzbanken bestimmt Unter diesen DNA-Sequenzen war die CDC33-Sequenz 4 mal vertreten, während die *CMK2*-Sequenz 3 mal auftrat. CDC33 kodiert einen wesentlichen Teil des "cap binding complex" durch Binden der 5' cap-Struktur von mRNAs (Sonenberg, 1996). Die Sekretionseffizienz von Gam1p wurde mit der des ursprünglich Gam1 p sezemierenden Wirtsstammes verglichen und das sezernierte Produkt wurde auf seine Glykosylierung hin überprüft.

### Beispiel 3 (nicht erfindungsgemäß)

### Gam1p-Herstellung in Stämmen, die das CDC33-Gen aus S. cerevisiae überexprimieren

Der rekombinante Stamm MF9:pMF23 wurde mit dem Plasmid pCDC33, welches die *CDC33-*kodierende Sequenz aus S. cerevisiae unter der Kontrolle des *GAL1*-Promotors enthält, retransformiert. Der ursprüngliche Stamm und der retransformierte Stamm wurden in SG-Medium entweder ohne Tryptophan (*TRP1*-Selektion) oder ohne Tryptophan und ohne Leucin (*TRP1* und *LEU2d*-Selektion) in 20 ml bei 30°C wachsen gelassen, bis die stationäre Phase erreicht war.

Die von den rekombinanten Stämmen sezernierte Glukoamylaseaktivität wurde anhand der neu erworbenen Fähigkeit der Hefezellen, Stärke degradieren zu können, gemessen. Diese Zellen wurden daher auf Platten aufgebracht, die 0,5% lösliche Stärke (gemäß Zulkowski, Merck, Darmstadt) enthielten. Nach dem Zellwachstum wurden die Platten mit Iodkristallen jodiert. Die Menge an sezernierter Glukoamylase wurde aus dem Durchmesser der farblosen Höfe auf dem rot gefärbten Hintergrund bestimmt. Für eine genauere Bestimmung der Glukoamylaseaktivitäten verschiedener Stämme wurden diese in die wie oben beschriebenen Medium wachsen gelassen und die Glukosebildung aus einem Stärkesubstrat gemessen (Dohmen et al., 1990; Gellissen et al., 1991). Für die Glukosebestimmung wurden die Komponenten des "glucose test kits" (Merck, Darmstadt) gemäß den Instruktionen des Herstellers verwendet. Glukoamylase-Aktivität wurde in Proben der Kulturüberstände und Zellwandfraktionen gemessen. Zusätzlich wurde die Enzym-Aktivität in Gesamtzellextrakten bestimmt, welche aus Zellen gewonnen wurden, die sich in der exponentiellen Wachstumsphase befanden. Die Aktivitäten wurden jeweils in mindestens drei unabhängigen Messungen bestimmt.

Überexpression des *CDC33*-Gens führte zu einem 2,5-fachen Anstieg in der Produktion an sezernierter Glukoamylase im Falle von *TRP1*-Selektion und zu einem 7-fachen Anstieg im Falle von *LEU2d*-Selektion (siehe Abb. 3).

| Stamm | Selektion | OD₆₀₀ | Aktivität im Überstand (Einheitenf/l) | Aktivität in der Zellwandfraktion (Einheiten/l) | Aktivität im Gesamtzellextrakt (Einheiten x10⁻³/ g Protein |
|---|---|---|---|---|---|
| MF9:pMF23 | *TRP1* | 5,0 | 122.0±18,6 | 256,3±14,9 | 54,7±8,4 |
| MF9:pMF23: *pCDC33* | *TRP1* | 5,0 | 292,9±34,5 | 237,1±15,0 | 53,2±2,5 |
| *"* | *LEU2d* | 4,5 | 841,8±12,0 | 350,1±27,7 | n.b. |

| | | | | | |
|---|---|---|---|---|---|
| n.b. = nicht bestimmt | | | | | |

### Beispiel 4

### Gam1p-Produktion von Stämmen, die das S. cerevisiae CMK2-Gen überexprimieren:

Der rekombinante Stamm MF9:pMF23 wurde mit dem Plasmid pCMK2, welches die *CMK2*-kodierende Sequenz aus S. cerevisiae unter der Kontrolle des *GAL1*-Promotors enthält, wie oben beschrieben retransformiert. Der ursprüngliche Stamm und der retransformierte Stamm wurden in SG-Medium entweder ohne Tryptophan (TRP1-Selektion) oder ohne Tryptophan und ohne Leucin (*TRP1* und *LEU2d-* Selektion) in 50 ml bei 30°C wachsen gelassen, bis die stationäre Phase erreicht war. Die Glukoamylase-Aktivität wurde wie vorher beschrieben in Proben der Kulturüberstände und der Zellwandfraktionen gemessen. Zusätzlich wurde Enzymaktivität in Proben von Gesamtzellextrakten bestimmt, die aus Zellen gewonnen wurden, welche sich in der stationären Phase befanden. Die Aktivitäten wurden jeweils in mindestens drei unabhängigen Messungen bestimmt.

Überexpression des *CMK2*-Gens führte zu einem 3-fachen Anstieg in der Produktion von sezernierter Glukoamylase im Fall von *TRP1*-Selektion und zu einem 6,4-fachen Anstieg im Falle von *LEU2d*-Selektion (siehe Abb. 4).

| Stamm | Selektion | OD₆₀₀ | Aktivität im Überstand (Einheiten/I) | Aktivität in der Zellwandfraktion (Einheiten/I) | Aktivität im Gesamtzellextrakt (Einheiten x10⁻³/ g Protein |
|---|---|---|---|---|---|
| MF9:pMF23 | *TRP1* | 5,0 | 122.0±18,6 | 256,3±14,9 | 54,7±8,4 |
| MF9:pMF23: pCMK2 | *TRP1* | 5,0 | 341,8±5,0 | 254,4±24,6 | 56,1±15,3 |
| " | *LEU2d* | 4,5 | 781,4±12,0 | 470,4±19,7 | n.b. |

| | | | | | |
|---|---|---|---|---|---|
| n.b. = nicht bestimmt | | | | | |

### Beispiel 5

### Ausmaß der Glykosylierung von Gam1p in rekombinanten S. cerevisiae-Stämmen, die CDC33 und CMK2-Gene überexprimieren

Das Gam1p-Protein ist ein Enzym mit einem Molekulargewicht > 140kDa. Das errechnete Molekulargewicht der Aminosäuresequenz ist 104 kDa. Der Unterschied zwischen apparentem und errechnetem Molekulargewicht ist auf die Anwesenheit von N- und O-Glykosylierung zurückzuführen. Die Behandlung von Gam1p mit dem Enzym PNGase F, das N-gekoppelte Zuckereinheiten spezifisch entfernt, führt zu einer Reduktion des Molekulargewichtes auf 120 kDa. Der verbleibende Unterschied zwischen 120 kDa und dem errechneten Molekulargewicht von 104 kDa ist auf O-gekoppelte Zuckereinheiten zurückzuführen. In diesem Beispiel wird das in dem ursprünglichen S. cerevisiae-Stamm hergestellte Gam1p-Protein mit dem in *CDC33-* und *CMK2*-Supertransformanden hergestellten Gam1p-Protein verglichen. Für diesen Zweck wurde eine Glukoamylase mit einem C-terminalen HA-Marker für die immunologische Identifizierung der heterologen Enzyme aus Zellfraktionen mit Hilfe der Western Blot-Analyse hergestellt. Durch schonende Homogenisierung wurden Zellextrakte hergestellt, und Zellfraktionen durch Zentrifugation in einem Saccharose-Dichte-Gradienten erhalten. Mit Hilfe von Marker-Enzymen, die spezifisch für bestimmte subzelluläre Fraktionen sind, wurden verschiedene zelluläre Kompartimente identifiziert (siehe Abb. 2). Die Anwesenheit und die Größe des heterologen Gam1p-Proteins wurde durch SDS-PAGE gemäß Laemmli bestimmt (Laemmli, 1970). Die aufgetrennten Polypeptide wurden auf PVDF-Membranen (Schleicher und Schuell) transferiert und mit spezifischen Antikörpern gegen das HA-Epitop (BabCo) behandelt. In den Stämmen, die das CMK2-Gen überexprimierten, bestand die Population an Gam1p-Proteinen aus Molekülen mit einem Molekulargewicht von 140 kDa und 104 kDa. Daraus wird ersichtlich, daß die Expression des CMK2-Gens zur Erzeugung von rekombinanten Proteinen beiträgt, deren Molekulargewicht dem Molekulargewicht der nicht modifizierten Aminosäurekette entspricht. Dieser Effekt wurde in *CDC33*-überexprimierenden Stämmen nicht beobachtet.

Diese Ergebnisse zeigen, daß CMK2-Überexpression sowohl zu einer Verbesserung der Sekretion als auch zu einer Reduktion der Glykosylierung führt. Überexpression von *CDC33* führt dagegen ausschließlich zu einem Anstieg der Sekretion. Das Ausmaß der Glykosylierung wird in diesem Fall nicht beeinflußt (siehe Abb. 5).

### Beispiel 6

### Ausmaß der Glykosylierung einer rekombinanten Phytase in H. polymorpha-Stämmen, die das aus S. cerevisiae stammende CMK2-Gen überexprimieren

Der Einfluß des *CMK2*-Gens auf die Glykosylierung von heterologen Proteinen wurde in einem Phytase-herstellenden H. polymorpha-Stamm untersucht, wie von Mayer et al., 1999, beschrieben. Der Stamm enthält 40 in das Genom integrierte Kopien eines Phytase-Expressionsplasmids, in dem die kodierende Sequenz für Phytase an ein aus H. polymorpha stammendes FMD-Promotorelement zur Expressionskontrolle fusioniert ist. Die betreffende Phytase-Variante besteht aus einer Aminosäurekette mit einem errechneten Molekulargewicht von 55 kDa. Hyperglykosylierung führt zu Proteinen in einem Bereich zwischen 60 kDa und 110 kDa. Der Produktionsstamm wurde mit einem Plasmid, welches das aus S. cerevisiae stammende *CMK2*-Gen entweder unter der Kontrolle des ebenfalls aus S. cerevisiae stammenden *GAL1*-Promotors oder unter der Kontrolle des das H. Polymorpha stammenden FMD-Promotors trägt, supertransformiert. Beispiele für Anwendungen des FMD-Promotors sind in EP 299108 veröffentlicht. Die resultierenden Supertransformanden wurden auf das Ausmaß an Glykosylierung der sezernierten Phytase durch vergleichende SDS-PAGE untersucht. In beiden Fällen wurde gefunden, daß die Hyperglykosylierung reduziert war. Anstatt einer Vielzahl von Proteinbanden mit unterschiedlichen Molekulargewichten wurden Banden mit distinkten Größen sichtbar (Fig. 6).

### Zitierte Literatur:

- Altmann und Trachsel, Nuc., Acid Res. 17, 5923 (1989)
- Ausubel et al. (eds), Current protocols in molecular biology. Wiley & Sons, New York (1987)
- Beggs, Molecular genetics in yeast, von Wettstein et al. eds., Munksgaard, Copenhagen (1981)
- Brenner et al., Mol. Cell. Biol. 8, 3556 (1988)
- Dohmen, Doktorarbeit, Heinrich-Heine-Universität Düsseldort (1989)
- Dohmen et al., Gene 95, 111 (1990)
- Dohmen et al., J. Biol. Chem. 270, 18099 (1995)
- Erhart und Hollenberg, J. Bact. 156, 625 (1983)
- Gellissen et al., Biotechnology 9, 291 (1991)
- Gellissen und Hollenberg, Gene 190, 87 (1997)
- Giga-Hama und Kumagai, "Foreign gene expression in fission yeast Schizosaccharomyces pombe", Springer, Berlin (1997)
- Hinnen et al., Gene expression in recombinant microorganisms, Smith, ed., Marcel Dekker, N.Y., 121 (1994)
- Hollenberg und Gellissen, Curr. Opin. Biotechnol. 8, 554 (1997)
- Johnston & Davis, Mol.Cell. Biol. 4 (8), 1440 (1984)
- Laemmli U, Nature 227, 680 (1970)
- Liu et al., Genetics 132, 665 (1992)
- Mayer et al., Biotechnol. Bioeng. 63, S. 373-381 (1999)
- Ohya et al., J. Biol. Chem. 266, 12784 (1991)
- Pausch et al., EMBO J. 10, 1511 (1991)
- Pearson und Lipman, Proc. Natl. Acad. Sci. USA 85, 2444 (1988)
- Sakei et al., Genetics 129, 499 (1988)
- Sambrook, Fritsch & Maniatis, Molecular Cloning - A Laboratory Handbook, Cold Spring Harbor Laboratory Press (1989)
- Shulman, Curr. Opin. Cell Biol. 5, 247 (1993)
- Shusta et al., Nature/Biotechnology 116, 773 (1998)
- Sikorski & Hieter, Genetics 122, 19 (1989)
- Smith et al., Science 229, 1219 (1985)
- Sonenberg, Translational control, Mathews et al. eds, Cold Spring Harbour, N.Y. (1996)
- WO98/13473

## Patentansprüche

1. Verfahren zum Herstellen eines rekombinanten Proteins wobei die Sekretion des rekombinanten Proteins gesteigert wird, umfassend die folgenden Schritte:
a) Exprimieren einer für das rekombinante Protein kodierenden Nukleinsäure in einer geeigneten Wirtszelle zusammen mit einer für ein Polypeptid mit der biologischen Aktivität einer Ca/Calmodulinabhängigen Proteinkinase (CaM-Kinase) kodierenden Nukleinsäure, wobei die für ein Polypeptid mit der biologischen Aktivität von CaM-Kinase kodierende Nukleinsäure unter der Kontrolle eines induzierbaren Promotors P1 steht;
b) Gewinnen des Proteins,
wobei die Nukleinsäure, die für ein Polypeptid mit der biologischen Aktivität einer CaM-Kinase kodiert, ausgewählt ist aus der folgenden Gruppe:
i) einer Nukleinsäure mit der CMK2-Gensequenz, die für CaM-Kinase aus Saccharomyces cerevisiae kodiert;
ii) einer durch Degeneration des genetischen Codes, durch Deletion, Insertion und/oder Addition von der in i) genannten Nukleinsäure abgeleiteten Nukleinsäure, die für ein Polypeptid mit der biologischen Aktivität einer CaM-Kinase kodiert;
iii) einem Fragment einer der in i) bis ii) genannten Nukleinsäuren, das für ein Polypeptid mit der biologischen Aktivität einer CaM-Kinase kodiert.

2. Das Verfahren nach Anspruch 1, **dadurch gekennzeichnet, dass** die geeignete Wirtszelle eine Pflanzenzelle, Tierzelle, Hefezelle, Pilzzelle oder Schleimpilzzelle ist.

3. Verfahren nach Anspruch 2, **dadurch gekennzeiehnet, dass** die Tierzelle eine Säuger- oder Insektenzelle ist.

4. Verfahren nach Anspruch 2, **dadurch gekennzeichnet, dass** die Hefezelle eine Zelle der Gattung Saccharomyces, Schizosaccharomyces, Kluyveromyces, Hansenula, Pichia, Schwanniomyces, Candida oder Yarrowia ist.

5. Verfahren nach Anspruch 2, **dadurch gekennzeichnet, dass** die Pilzzelle eine Zelle der Gattung Aspergillus, Neurospora, Rhizopus oder Trichoderma ist.

6. Verfahren nach Anspruch 2, **dadurch gekennzeichnet, dass** die Schleimpilzzelle eine Zelle der Gattung Dictvostelium ist

7. Verfahren nach mindestens einem der Ansprüche 1 bis 6, **dadurch gekennzeichnet, dass** der Promotor P1 ein starker Promotor ist.

8. Verfahren nach mindestens einem der Ansprüche 1 bis 7, **dadurch gekennzeichnet, dass** das sekretorische Protein aus der folgenden Gruppe ausgewählt ist: Phytase, Glukoamylase, Phosphatase, Wachstumsfaktoren.

9. Verwendung mindestens einer Nukleinsäure zum Steigern der Sekretion eines rekombinanten sekretorischen Proteins aus einer Wirtszelle, **dadurch gekennzeichnet, dass** die Nukleinsäure ausgewählt ist aus der folgenden Gruppe:
i) einer Nukleinsäure mit der CMK2-Gensequenz, die für CaM-Kinase aus Saccharomyces cerevisiae kodiert;
ii) einer durch Degeneration des genetischen Codes, durch Deletion, Insertion und/oder Addition von der in i) genannten Nukleinsäure abgeleiteten Nukleinsäure, die für ein Polypeptid mit der biologischen Aktivität einer CaM-Kinase kodiert;
iii) einem Fragment einer der in i) bis ii) genannten Nukleinsäuren, das für ein Polypeptid mit der biologischen Aktivität einer CaM-Kinase kodiert.

10. Verwendung einer Nukleinsäure nach Anspruch 9, **dadurch gekennzeichnet, dass** die Nukleinsäure in einen Vektor integriert ist.

11. Wirtszelle, **dadurch gekennzeichnet, dass** sie eine durch ein rekombinantes Verfahren in die Zelle eingeschleuste Nukleinsäure, die das herzustellende Protein kodiert, sowie eine durch ein rekombinantes Verfahren in die Zelle eingeschleuste Nukleinsäure enthält, die aus der folgenden Gruppe ausgewählt ist:
i) einer Nukleinsäure mit der CMK2-Gensequenz, die für CaM-Kinase aus Saccharomyces cerevisiae kodiert;
ii) einer durch Degeneration des genetischen Codes, durch Deletion, Insertion und/oder Addition von der in i) genannten Nukleinsäure abgeleiteten Nukleinsäure, die für ein Polypeptid mit der biologischen Aktivität einer CaM-Kinase kodiert;
iii) einem Fragment einer der in i) bis ii) genannten Nukleinsäuren, das für ein Polypeptid mit der biologischen Aktivität einer CaM-Kinase kodiert.

12. Kit, **dadurch gekennzeichnet, dass** er umfasst:
(a) mindestens einen Expressionsvektor, umfassend Nukleinsäuren, wobei die Nukleinsäuren für Polypeptide mit der biologischen Aktivität einer CaM-Kinase und eIF4E kodieren, wobei die für ein Polypeptid mit der biologischen Aktivität von eIF4E kodierende Nukleinsäure ausgewählt aus der folgenden Gruppe:
i) einer Nukleinsäure mit der CDC33-Gensequenz, die für eIF4E aus Saccharomyces cerevisiae kodiert;
ii) einer Nukleinsäure, die für ein Homolog von eIF4E mit einer Homologie von mindestens 90 % und mit der biologischen Aktivität von eIF4E kodiert;
iii) einer durch Degeneration des genetischen Codes, durch Deletion, Insertion und/oder Addition von der in i) oder ii) genannten Nukleinsäure abgeleiteten Nukleinsäure, die für ein Polypeptid mit der biologischen Aktivität von eIF4E kodiert
iv) einem Fragment einer der in i) bis iii) genannten Nukleinsäuren, das für ein Polypeptid mit der biologischen Aktivität eines eIF4E kodiert; und wobei die für ein Polypeptid mit der biologischen Aktivität einer CaM-Kinase kodierende Nukleinsäure ausgewählt ist aus der Gruppe:
i) einer Nukleinsäure mit der CMK2-Gensequenz, die für CaM-Kinase aus Saccharomyces cerevisiae kodiert;
ii) einer durch Degeneration des genetischen Codes, durch Deletion und/oder Addition von der in i) genannten Nukleinsäure abgeleiteten Nukleinsäure, die für ein Polypeptid mit der biologischen Aktivität einer CaM-Kinase kodiert;
iii) einem Fragment einer der in i) bis ii) genannten Nukleinsäuren, das für ein Polypeptid mit der biologischen Aktivität einer CaM-Kinase kodiert;
(b) eine zur Sezernierung und/oder Glykosylierung von Proteinen geeignete Wirtszelle.

13. Kit, **dadurch gekennzeichnet, dass** er umfasst:
(a) eine Wirtszelle, die eine durch ein rekombinantes Verfahren in die Zelle eingeschleuste Nukleinsäure enthält, die für ein Polypeptid mit der biologischen Aktivität einer CaM-Kinase kodiert und aus der folgenden Gruppe ausgewählt ist:
i) einer Nukleinsäure mit der CMK2-Gensequenz, die für CaM-Kinase aus Saccharomyces cerevisiae kodiert;
ii) einer durch Degeneration des genetischen Codes, durch Deletion, Insertion und/oder Addition von der in i) genannten Nukleinsäure abgeleiteten Nukleinsäure, die für ein Polypeptid mit der biologischen Aktivität einer CaM-Kinase kodiert;
iii) einem Fragment einer der in i) bis ii) genannten Nukleinsäuren, das für ein Polypeptid mit der biologischen Aktivität einer CaM-Kinase kodiert;
(b) einen leeren Expressionsvektor, der zum Hineinklonieren einer Nukleinsäure geeignet ist, welche ein rekombinantes Protein kodiert.

## Claims

1. Method for producing a recombinant protein, wherein the secretion of the recombinant protein is increased, comprising the following steps:
a) expressing a nucleic acid coding for the recombinant protein in a suitable host cell, together with a nucleic acid coding for a polypeptide having the biological activity of a Ca/calmodulin-dependent protein kinase (CaM kinase), said nucleic acid coding for a polypeptide having the biological activity of a CaM kinase being under the control of an inducible promoter P1;
b) obtaining the protein,
wherein the nucleic acid coding for a polypeptide having the biological activity of a CaM kinase is selected from the following group:
i) a nucleic acid having the CMK2 gene sequence that is coding for CaM kinase from Saccharomyces cerevisiae;
ii) a nucleic acid derived from the nucleic acid set forth in i) by degeneration of the genetic code, by deletion, insertion and/or addition, said nucleic acid coding for a polypeptide having the biological activity of a CaM kinase;
iii) a fragment of one of the nucleic acids set forth in i) to ii), said fragment coding for a polypeptide having the biological activity of a CaM kinase.

2. Method according claim 1, **characterized in that** the suitable host cell is a plant cell, animal cell, yeast cell, fungal cell or slime fungus cell.

3. Method according to claim 2, **characterized in that** the animal cell is a mammalian cell or insect cell.

4. Method according to claim 2, **characterized in that** the yeast cell is a cell of the genus Saccharomyces, Schizosaccharomyces, Kluyveromyces, Hansenula, Pichia, Schwanniomyces, Candida or Yarrowia.

5. Method according to claim 2, **characterized in that** the fungal cell is a cell of the genus Aspergillus, Neurospora, Rhizopus or Trichoderma.

6. Method according to claim 2, **characterized in that** the slime fungus cell is a cell of the genus Dictyostelium

7. Method according to at least one of claims 1 to 6, **characterized in that** the promoter P1 is a strong promoter.

8. Method according to at least one of claims 1 to 7, **characterized in that** the secretory protein is selected from the following group: phytase, glucoamylase, phosphatase, growth factors.

9. Use of at least one nucleic acid to increase secretion of a recombinant secretory protein from a host cell, **characterized in that** the nucleic acid is selected from the following group:
i) a nucleic acid having the CMK2 gene sequence that is coding for CaM kinase from Saccharomyces cerevisiae;
ii) a nucleic acid derived from the nucleic acid set forth in i) by degeneration of the genetic code, by deletion, insertion and/or addition, said nucleic acid coding for a polypeptide having the biological activity of a CaM kinase;
iii) a fragment of one of the nucleic acids set forth in i) to ii), said fragment coding for a polypeptide having the biological activity of a CaM kinase.

10. Use of a nucleic acid according to claim 9, **characterized in that** the nucleic acid is integrated into a vector.

11. Host cell, **characterized in that** it contains a nucleic acid coding for the protein that is to be produced, said nucleic acid being introduced into the cell by a recombinant process, as well as a nucleic acid that is introduced into the cell by a recombinant process, said nucleic acid being selected from the following group:
i) a nucleic acid having the CMK2 gene sequence that is coding for CaM kinase from Saccharomyces cerevisiae;
ii) a nucleic acid derived from the nucleic acid set forth in i) by degeneration of the genetic code, by deletion, insertion and/or addition, said nucleic acid coding for a polypeptide having the biological activity of a CaM kinase;
iii) a fragment of one of the nucleic acids set forth in i) to ii), said fragment coding for a polypeptide having the biological activity of a CaM kinase.

12. Kit, **characterized in that** it comprises:
(a) at least one expression vector which comprises nucleic acids, said nucleic acids coding for polypeptides having the biological activity of a CaM kinase and eIF4E, said nucleic acid coding for a polypeptide having the biological activity of eIF4E being selected from the following group:
i) a nucleic acid having the CDC33 gene sequence that is coding for eIF4E from Saccharomyces cerevisiae;
ii) a nucleic acid coding for a homologue of eIF4E having a homology of at least 90 % and having the biological activity of eIF4E;
iii) a nucleic acid derived from the nucleic acid set forth in i) or ii) by degeneration of the genetic code, by deletion, insertion and/or addition, said nucleic acid coding for a polypeptide having the biological activity of eIF4E;
iv) a fragment of one of the nucleic acids set forth in i) to iii), said fragment coding for a polypeptide having the biological activity of an eIF4E;
and wherein the nucleic acid coding for a polypeptide having the biological activity of a CaM kinase is selected from the group:
i) a nucleic acid having the CMK2 gene sequence that is coding for CaM kinase from Saccharomyces cerevisiae;
ii) a nucleic acid derived from the nucleic acid set forth in i) by degeneration of the genetic code, by deletion, insertion and/or addition, said nucleic acid coding for a polypeptide having the biological activity of a CaM kinase;
iii) a fragment of one of the nucleic acids set forth in i) to ii), said fragment coding for a polypeptide having the biological activity of a CaM kinase.
(b) a host cell suitable for secretion and/or glycosilation of proteins.

13. Kit, **characterized in that** it comprises:
(a) a host cell comprising a nucleic acid that has been introduced into the cell by a recombinant process, said nucleic acid coding for a polypeptide having the biological activity of a CaM kinase and being selected from the following group:
i) a nucleic acid having the CMK2 gene sequence that is coding for CaM kinase From Saccharomyces cerevisiae;
ii) a nucleic acid derived from the nucleic acid set forth in i) by degeneration of the genetic code, by deletion, insertion and/or addition, said nucleic acid coding for a polypeptide having the biological activity of a CaM kinase;
iii) a fragment of one of the nucleic acids set forth in i) to ii), said fragment coding for a polypeptide having the biological activity of a CaM kinase.
(b) an empty expression vector suitable for the cloning in of a nucleic acid coding for a recombinant protein.

## Revendications

1. Procédé pour produire une protéine recombinée, la sécrétion de la protéine recombinée étant alors accrue, comprenant les étapes suivantes consistant à :
a) exprimer un acide nucléique codant pour la protéine recombinée dans une cellule hôte adaptée conjointement avec un acide nucléique codant pour un polypeptide ayant l'activité biologique d'une protéine-kinase Ca/calmoduline-dépendante (CaM-kinase), l'acide nucléique codant pour un polypeptide ayant l'activité biologique de la CaM-kinase étant sous le contrôle d'un promoteur inductible P1;
b) obtenir la protéine ;
l'acide nucléique codant pour un polypeptide ayant l'activité biologique d'une CaM-kinase étant choisi dans le groupe suivant :
i) un acide nucléique avec la séquence génique de la CMK2 codant pour la CaM-kinase de *Saccharomyces cerevisiae ;*
ii) un acide nucléique dérivé de l'acide nucléique présenté au point i) par dégénérescence du code génétique, par délétion, insertion et/ou addition, l'acide nucléique codant pour un polypeptide ayant l'activité biologique d'une CaM-kinase;
iii) un fragment de l'un des acides nucléiques présentés aux points i) à ii), le fragment codant pour un polypeptide ayant l'activité biologique d'une CaM-kinase.

2. Procédé selon la revendication 1, **caractérisé en ce que** la cellule hôte adaptée est une cellule végétale, une cellule animale, une cellule de levure, une cellule fongique ou une cellule de myxomycetes.

3. Procédé selon la revendication 2, **caractérisé en ce que** la cellule animale est une cellule de mammifère ou une cellule d'insecte.

4. Procédé selon la revendication 2, **caractérisé en ce que** la cellule de levure est une cellule du genre *Saccharomyces, Schizosaccharomyces, Kluyveromyces, Hansenula, Pichia, Schwanniomyces, Candida* ou *Yarrowia.*

5. Procédé selon la revendication 2, **caractérisé en ce que** la cellule fongique est une cellule du genre *Aspergillus, Neurospora, Rhizopus* ou *Trichoderma.*

6. Procédé selon la revendication 2, **caractérisé en ce que** la cellule de myxomycetes est une cellule du genre *Dictyostelium.*

7. Procédé selon au moins l'une des revendications 1 à 6, **caractérisé en ce que** le promoteur P1 est un promoteur puissant.

8. Procédé selon au moins l'une des revendications 1 à 7, **caractérisé en ce que** la protéine sécrétoire est choisie dans le groupe suivant constitué par la phytase, la glucoamylase, la phosphatase et les facteurs de croissance.

9. Utilisation d'au moins un acide nucléique pour accroître la sécrétion d'une protéine sécrétoire recombinée provenant d'une cellule hôte, **caractérisée en ce que** l'acide nucléique est choisi dans le groupe suivant constitué par :
i) un acide nucléique avec la séquence génique de la CMK2 codant pour la CaM-kinase issue de *Saccharomyces cerevisiae ;*
ii) un acide nucléique dérivé de l'acide nucléique présenté au point i) par dégénérescence du code génétique, par délétion, insertion et/ou addition, l'acide nucléique codant pour un polypeptide ayant l'activité biologique d'une CaM-kinase ;
iii) un fragment de l'un des acides nucléiques présentés aux points i) à ii), le fragment codant pour un polypeptide ayant l'activité biologique d'une CaM-kinase.

10. Utilisation d'un acide nucléique selon la revendication 9, **caractérisée en ce que** l'acide nucléique est intégré dans un vecteur.

11. Cellule hôte, **caractérisée en ce qu'**elle contient un acide nucléique introduit dans la cellule par un procédé de recombinaison, l'acide nucléique codant pour la protéine produite ainsi qu'un acide nucléique introduit dans la cellule par un procédé de recombinaison, l'acide nucléique étant choisi dans le groupe suivant comprenant :
i) un acide nucléique avec la séquence génique de la CMK2 codant pour la CaM-kinase de *Saccharomyces cerevisiae* ;
ii) un acide nucléique dérivé de l'acide nucléique présenté au point i) par dégénérescence du code génétique, par délétion, insertion et/ou addition, l'acide nucléique codant pour un polypeptide ayant l'activité biologique d'une CaM-kinase ;
iii) un fragment de l'un des acides nucléiques présentés aux points i) à ii), le fragment codant pour un polypeptide ayant l'activité biologique d'une CaM-kinase.

12. Trousse, **caractérisée en ce qu'**elle comprend :
(a) au moins un vecteur d'expression comprenant des acides nucléiques, les acides nucléiques codant pour des polypeptides ayant l'activité biologique d'une CaM-kinase et de elF4E, l'acide nucléique codant pour un polypeptide ayant l'activité biologique de elF4E étant choisi dans le groupe suivant constitué par :
i) un acide nucléique ayant la séquence génique du CDC33 codant pour elF4E de *Saccharomyces cerevisiae ;*
ii) un acide nucléique codant pour un homologue de elF4E présentant une homologie d'au moins 90 % et ayant l'activité biologique de elP4E ;
iii) un acide nucléique dérivé de l'acide nucléique présenté au point i) ou ii) par dégénérescence du code génétique, par délétion, insertion et/ou addition, l'acide nucléique codant pour un polypeptide ayant l'activité biologique de e1F4E ;
iv) un fragment d'un des acides nucléiques présentés aux points i) à iii), le fragment codant pour un polypeptide ayant l'activité biologique d'un elF4E ;
et l'acide nucléique codant pour un polypeptide ayant l'activité biologique d'une CaM-kinase étant choisi dans le groupe constitué par :
i) un acide nucléique avec la séquence génique de la CMK2 codant pour la CaM-kinase de *Saccharomyces cerevisiae* ;
ii) un acide nucléique dérivé de l'acide nucléique présenté au point i) par dégénérescence du code génétique par délétion, et/ou addition, l'acide nucléique codant pour un polypeptide ayant l'activité biologique d'une CaM-kinase ;
iii) un fragment de l'un des acides nucléiques présentés aux points i) à ii), le fragment codant pour un polypeptide ayant l'activité biologique d'une CaM-kinase ;
b) une cellule hôte adaptée pour la sécrétion et/ou la glycosylation des protéines.

13. Trousse, **caractérisée en ce qu'**elle comprend :
(a) une cellule hôte qui contient un acide nucléique introduit dans la cellule par un procédé de recombinaison, l'acide nucléique codant pour un polypeptide ayant l'activité biologique d'une CaM-kinase et choisi dans le groupe suivant constitué par :
i) un acide nucléique avec la séquence génique de la CMK2 codant pour la CaM-kinase de *Saccharomyces cerevisiae ;*
ii) un acide nucléique dérivé de l'acide nucléique présenté au point i) par dégénérescence du code génétique, par délétion, et/ou addition, l'acide nucléique codant pour un polypeptide ayant l'activité biologique d'une CaM-kinase ;
iii) un fragment de l'un des acides nucléiques présentés aux points i) à ii), le fragment codant pour un polypeptide ayant l'activité biologique d'une CaM-kinase.
b) un vecteur d'expression vide qui est adapté au clonage d'un acide nucléique, lequel code pour une protéine recombinée.
